# EUROPEAN PATENT SPECIFICATION

(11) **EP 4 079 365 B1**
(45) Date of publication and mention of the grant of the patent: **11.03.2026**
(21) Application number: 22165299.3
(22) Date of filing: 30.03.2022
(51) Int. Cl.: A61M 25/01, A61B 18/14

(54) **ELECTROPHYSIOLOGY DEVICES WITH DEFLECTION DETECTION**
ELEKTROPHYSIOLOGIEVORRICHTUNGEN MIT ABLENKDETEKTION
DISPOSITIFS D'ÉLECTROPHYSIOLOGIE AVEC DÉTECTION DE DÉVIATION

(30) Priority: 31.03.2021 US 202117219545
(43) Date of publication of application: 26.10.2022
(73) Proprietor: BIOSENSE WEBSTER (ISRAEL) LTD., 2066717 Yokneam (IL)
(72) Inventor: TANG, Raymond Yue-Sing, Irvine, 92618 (US)
(74) Representative: Carpmaels & Ransford LLP

(56) References cited:
- US-A1- 2010 063 478
- US-A1- 2014 350 340
- US-A1- 2020 054 864
- US-B1- 6 272 371

## Description

### FIELD OF INVENTION

This invention relates to a guiding sheath which is especially suitable for guiding electrophysiology catheters, in particular, a deflectable guiding sheath.

### BACKGROUND

Guiding sheaths and electrophysiology (EP) catheters are well known for use in the diagnosis and/or treatment of various cardiovascular conditions, including atrial fibrillation and other abnormal heart rates known as arrhythmias. Catheterization of the human heart often necessitates having an elongated EP probe gain access to the heart chambers, via a femoral vein and the aorta. To provide variation in movement in such devices, guiding sheaths, catheters and the like may be steerable or deflectable with the use of one or more puller wires for maneuverability in the patient's vasculature. While control handles are aptly manipulated by operators to deflect these EP tools while inside patient's vasculature and heart, the occurrence and degree of deflection can be difficult to assess as times.

With ablation catheters, irrigation of the ablation tissue site provides a number of benefits, including cooling the ablated tissue and creating deeper and larger lesions. Irrigation fluid, such as saline, is delivered to the ablation site by a lumened tubing that passes the fluid typically from a remote IV bag and drip chamber to a luer hub connected to an irrigation tubing that extends through the control handle and the shaft of the EP device. As such, fluid from the IV bag typically drips into the drip chamber and as the IV bag is depleted and the reservoir of the drip chamber empties, there is the risk of air in the drip chamber passing into the lumened tubing, the irrigation tubing of the EP device and further into the patient's vasculature where the errant introduction of air can be fatal.

Accordingly, there is a desire for a guiding sheath or catheter that can provide an indication of deflection occurrence and direction and whether the deflection curve is tightening or relaxing, and also a drip chamber that automatically closes off the drip chamber outlet when the IV bag is depleted.

US2020/054864A1 describes a catheter tube comprising a sensor arranged to provide a sensor signal representative for a degree of bending of the catheter tube.

### SUMMARY OF THE INVENTION

The invention is as defined in the independent claims.

### SUMMARY OF THE DISCLOSURE

In some embodiments, a guiding sheath assembly, comprising an elongated shaft and a control handle. The control handle has a longitudinal axis and a control knob, and including a first shuttle configured for translation along the longitudinal axis in one direction in response to one manipulation of the control knob, and a second shuttle configured for translation along the longitudinal axis in another direction opposite to the one direction in response to another manipulation of the control knob. A first puller wire extends on one side of the shaft and is responsive to translation of the first shuttle to deflect the shaft toward the one side, the first puller wire having a first stop at its proximal end. A second puller wire extends on an other side of the shaft and is responsive to translation of the second shuttle to deflect the shaft toward the other side, the second puller wire having a second stop at its proximal end. The assembly further includes a first deflection sensor configured to generate a first signal in response to compression between the first shuttle and the first stop.

In some embodiments, the guiding sheath assembly further includes a second deflection sensor situated between the second shuttle and the second stop and configured to generate a second signal in response to compression between the second shuttle and the second stop.

In some embodiments, the first deflection sensor includes a piezoelectric pressure sensor.

In some embodiments, the first signal includes a voltage signal.

In some embodiments, a guiding sheath assembly has an elongated shaft and a control handle. The control handle has a longitudinal axis, and includes a rotatable shaft configured for rotation about a longitudinal axis of the control handle; a first shuttle configured for translation along the longitudinal axis in one direction in response to rotation of the rotatable shaft, the first shuttle having a first plurality of teeth; a pinion in engagement with the first plurality of teeth, the pinion configured for rotation about an axis generally perpendicular to the longitudinal axis in response to the translation of the first shuttle; and a second shuttle having a second plurality of teeth in engagement with the pinion, the second shuttle configured for translation along the longitudinal axis in an other direction opposite to the one direction in response to rotation of the pinion. The assembly also includes a first puller wire extending on one side of the shaft and having a proximal portion responsive to translation of the first shuttle in a proximal direction, the first puller wire having a first stop at its proximal end; a second puller wire extending on an other side of the shaft and having a proximal portion response to translation of the second shuttle in the proximal direction, the second puller wire having a second stop at its proximal end; and a first deflection sensor situated between the first shuttle and the first stop and configured to generate a first signal responsive to compression when the first shuttle is actuated to deflect the elongated shaft in one direction.

In some embodiments, the guiding sheath assembly further includes a second deflection sensor situated between the second shuttle and the second stop and configured to generate a second signal responsive to compression of the second stop when the second shuttle is actuated to deflect the elongated shaft in an other direction.

In some embodiments, the first deflection sensor includes a piezoelectric pressure sensor.

In some embodiments, the first signal includes a voltage signal.

In some embodiments, an electrophysiology system includes a guiding sheath assembly and a controller. The assembly includes an elongated shaft; a control handle proximal of the shaft, the control handle having a longitudinal axis and a deflection control knob, and including a shuttle configured for translation along the longitudinal axis in response to manipulation of the control knob; a puller wire extending along the shaft and responsive to translation of the shuttle to deflect the elongated shaft, the puller wire having a stop at its proximal end; and a deflection sensor configured to generate a signal in response to compression between the shuttle and the stop in when the elongated shaft is deflected. The controller includes a processor configured to receive the signal and to perform the acts of measuring a voltage from the signal, and determining an occurrence of deflection based on the voltage measured.

In some embodiments, the processor is further configured to perform the act of determining a degree of deflection based on the voltage measured.

In some embodiments, the console includes a memory configured to store an index correlating predetermined voltages and predetermined degrees of deflection, and the processor is further configured to access the index to determine the degree of deflection based on the voltage measured.

In some embodiments, the deflection sensor includes a piezoelectric pressure sensor.

In some embodiments, the system includes an indicator responsive to the controller and configured to provide a cue to a user indicative of the occurrence of deflection based on the voltage measured.

In some embodiments, the indicator is configured to provide a visual cue.

In some embodiments, the indicator is configured to provide an audio cue.

In some embodiments, an electrophysiology catheter has an elongated shaft; and a control handle proximal of the shaft, the control handle having a longitudinal axis and a control knob. The catheter further includes a puller wire extending through the shaft and having a proximal end, a rocker responsive to the control knob to deflect the elongated shaft, the rocker having a pulley member around which the puller wire extends, a stop affixed to the proximal end and anchored to the control handle, and a strain gauge affixed to the stop and configured to generate a signal in response to strain of the stop.

In some embodiments, the control handle also includes a second puller wire extending through an other side of the shaft and having a second proximal end, wherein the rocker has a second pulley member around which the second puller wire extends. The control handle also has a second stop affixed to the second proximal end and anchored to the control handle, and a second strain gauge affixed to the second stop and configured to generate a second signal in response to strain of the second stop.

In some embodiments, the signal includes a voltage signal.

In some embodiments, an electrophysiology catheter includes an elongated shaft and a control handle. The control handle has a longitudinal axis and a control knob, and further includes a rocker responsive to the control knob to deflect the elongated shaft, the rocker having a pulley member, a tensile member having a distal portion extending through the shaft and a proximal portion extending around the pulley member, a connector extending between the distal and proximal portions of the tensile member, and a strain gauge affixed to the connector and configured to generate a signal in response to strain of the connector.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 is a top plan view of a guiding sheath including a control handle, in accordance with an embodiment.
FIG. 2 is a longitudinal cross-sectional view of a control handle of FIG. 1.
FIG. 3 is an exploded view of the control handle of FIG. 1, with a housing removed.
FIG. 4 is a longitudinal cross-sectional view of a distal portion of the control handle of FIG. 1, including a control knob.
FIG. 5 is a perspective view of the control handle of FIG. 1, with the housing removed.
FIG. 6A is a top plan view of a neutral indicator with first and second members engaged, in accordance with one embodiment.
FIG. 6B is a top plan view of the neutral indicator of FIG. 6A with the first and second members disengaged.
FIG. 7A and FIG. 7B are top plan views of the interior of a bi-directional guiding sheath control handle with first and second shuttle members and deflection sensors, in accordance with an embodiment.
FIG. 8A and FIG. 8B are detailed side elevational views of a deflection sensor of FIG. 7A and FIG. 7B, situated between a shuttle member and a stop.
FIG. 9 is a depiction of an EP system including an EP device (e.g., a guiding sheath or a catheter) and a console, in use in a patient as manipulated by an operator, according to one embodiment.
FIG. 10 is a top plan view of the interior of a bi-directional control handle of an EP catheter, according to one embodiment.
FIG. 11A, FIG. 11B and FIG. 11C are simplified depictions of the control handle of FIG. 10 in a neutral configuration, deflected in one direction, and deflected in another direction, respectively.
FIG. 12 is a detailed top plan view of a strain gauge on a tensile member connector, according to one embodiment.
FIG. 13 is a side cross-sectional view of a thumb control handle, according to one embodiment.

### DETAILED DESCRIPTION OF THE INVENTION

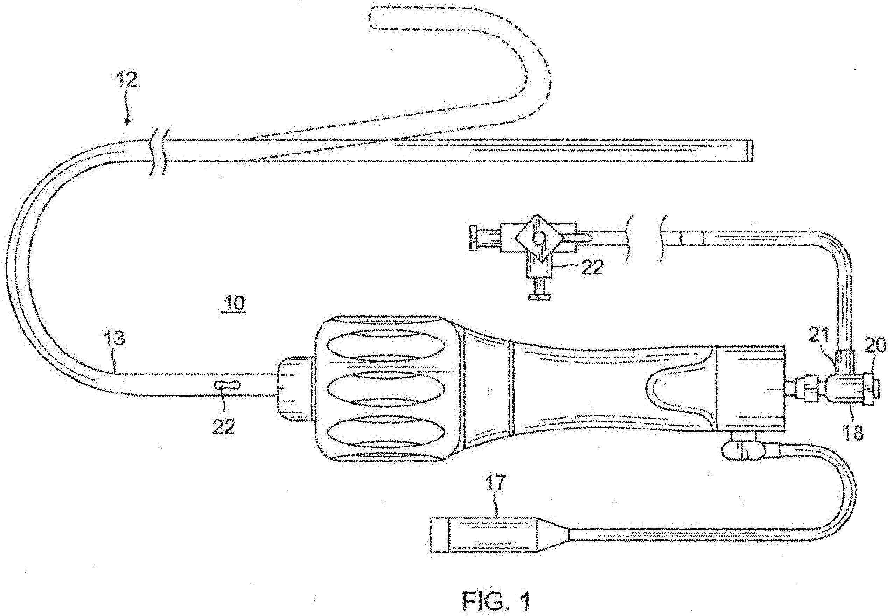

Referring to FIG. 1, in some embodiment of present invention, a guiding sheath assembly 10 includes an elongated and flexible sheath 12, and a control handle 16 proximal of sheath 12. The sheath 12 includes a proximal section 13 and a distal deflection section 14. The control handle 16 may be connected to an electrical connector 17 for transmitting electrical signals, as sensed by one or more ring electrodes 19 carried on the sheath 12, including, for example, the deflection section 14. Also attached to the control handle 16, as shown in FIG. 1, is a hemostatic valve 18 adapted to receive a catheter (not shown) that can be advanced through a center lumen 22 of the guiding sheath assembly 10 (fix FIG. 1). The hemostatic valve 18 also has side port 21 terminating in a luer hub, such as a two-way stop cock 23, for connection to one or more fluid sources (not shown) for providing fluid into and through the lumen 22 of the guiding sheath assembly 10.

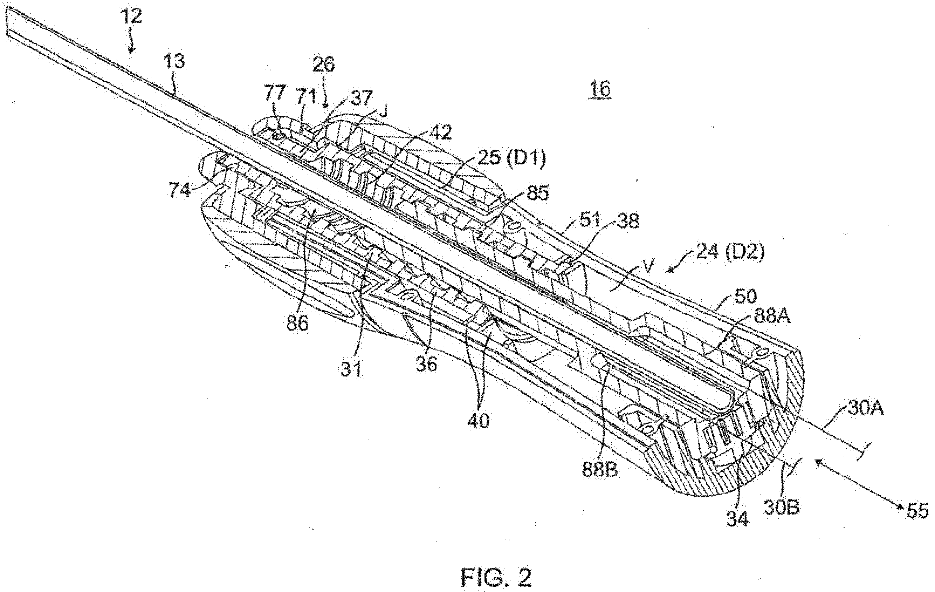

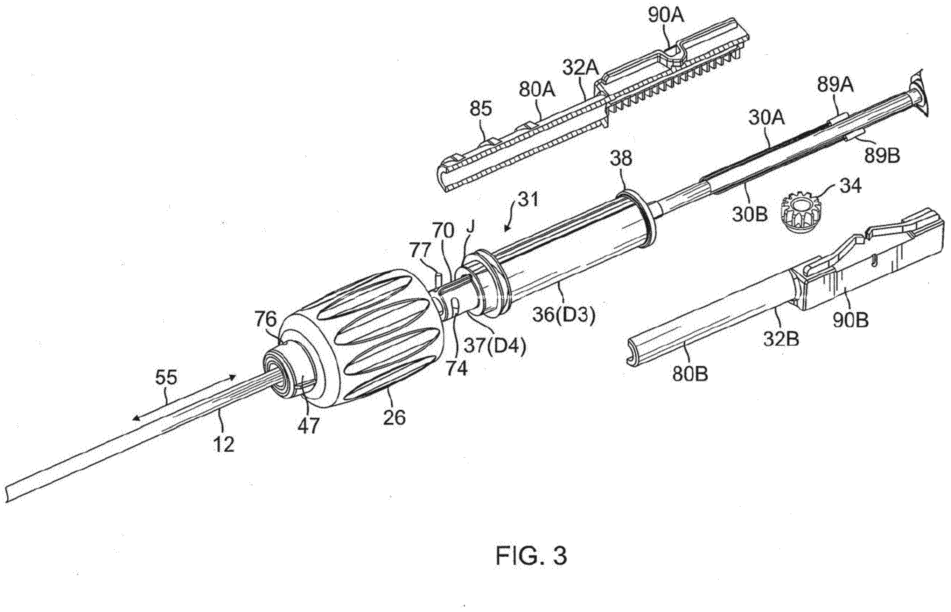

As shown in FIG. 2 and FIG. 3, the control handle 16 includes an elongated, generally cylindrical main body 24 with a narrower distal portion or stem 25, and a distal rotational control knob 26 mounted on the distal portion stem 25. The main body 24 has an outer shell-half member formed to define an interior volume V and whose edges 51 meet along a longitudinal seam. The distal stem 25 of the main body has a smaller outer diameter D1 compared to the outer diameter D2 of a proximal portion of the main body 24. The control knob 26 is configured for rotation by a user's thumb and forefinger when the user is grasping the main body 24 of the control handle 16. To enable deflection of the deflection section 14 of the guiding sheath 12 via first and second puller wires 30A and 30B, the control handle 16 includes in its interior volume V a rotatable shaft 31, first and second shuttles 32A and 32B, and a pinion 34. The rotatable shaft 31 is responsive to the control knob 26 in driving first shuttle 32A to move linearly along a longitudinal axis 55 in a first direction, and the pinion 34 couples the second shuttle 32B to the first shuttle 32A such that the second shuttle 32B moves linearly along the longitudinal axis in a second direction opposition to the first direction. With proximal ends of the first and second puller wires 30A and 30B anchored, or at least coupled, to the first and second shuttles 32A and 32B, respectively, such coupled and opposite translational movement of the first and second shuttles actuate the first and second puller wires for bi-directional deflection of the deflection section 14 of the guiding sheath 12.

The rotatable shaft 31 has a main proximal section 36 with an outer diameter D3, a shorter distal section 37 with an outer diameter D4, and a step junction J therebetween between sections 36 and 37. In the illustrated embodiment, the diameter D3 is greater than the diameter D4, but it is understood that the two diameters may be generally equal or the diameter D4 may be greater than the diameter D3. As better seen in FIG. 2, the rotatable shaft 31 is situated relative to main body 24 of the control handle 16 such that its proximal section 36 extends through both the main body 24 and the distal stem 25 of the control handle 16 and past a distal end of the distal stem 25, with the junction J and the distal section 37 being distal of the distal stem 25 of the main body 24 so that the distal section 37 is not surrounded by the distal stem 25. The rotatable shaft 31 is connected and affixed at its proximal end to the main body 24 by a proximal outer circumferential lip 38 that engages with an inner circumferential slot defined between circumferential flanges 40 formed in the interior volume V of the main body 24.

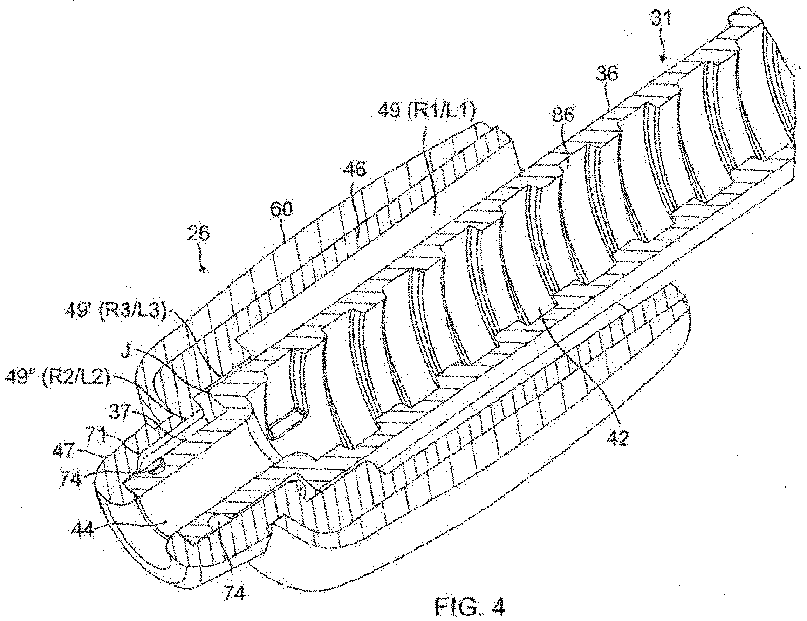

With reference to FIG. 4, the rotatable shaft 31 is hollow having an interior passage 42. The passage 42 is in communication with a distal inlet 44 whose diameter is merely slightly greater than the diameter of the guiding sheath 12. The passage 42 is threaded and has a diameter to accommodate both the guiding sheath 12 and the shuttles 32A and 32B circumferentially surrounding the guiding sheath 12, as discussed below in further detail.

The control knob 26, which is mounted on the distal stem 25 of the main body 24 of the control handle 16 and the rotatable shaft 31, has a main proximal portion 46 and a short distal end portion 47. The control knob 26 is generally cylindrical with a longitudinal hollow interior that extends through its entire length. The hollow interior has a main proximal section 49, a mid-section 49' and a distal section 49". The main proximal section 49 of the hollow interior is defined by a greater first radius R1 and a greater first length L1 to accommodate and circumferentially surround the guiding sheath 12 and the shuttles 32A and 32B. The distal section 49" of the hollow interior is defined by a lesser second radius R2, where R1>R2, and a shorter second length L2, where L1>L2, to accommodate and circumferentially surround the guiding sheath 12 and the distal section 37 of the rotatable shaft 31. The mid-section 49' of the hollow interior is defined by a third radius R3, where R1>R3>R2, and a third length L3, where L1>L3, to accommodate and circumferentially surround the guiding sheath 12 and the junction J of the rotatable shaft 31. A friction-inducing cover 60 may be mounted on an outer surface of the control knob 26 for the user's ease and comfort in manipulating and rotating the control knob relative to the main body 24 of the control handle 16.

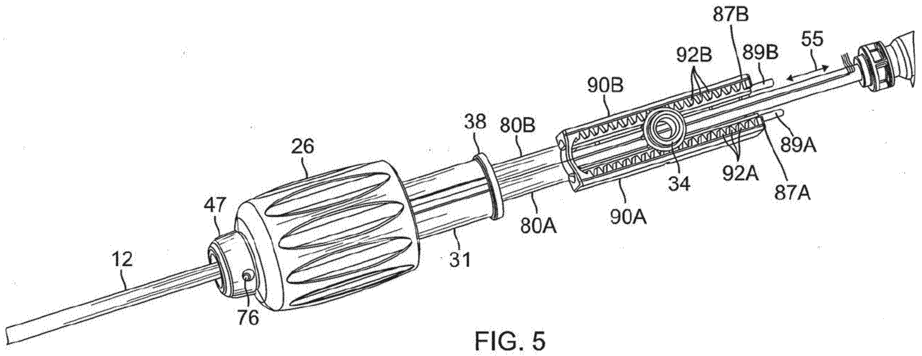

To rotationally couple the rotatable shaft 31 to the control knob 26, an outer surface of the distal section of the shaft has a longitudinal ridge 70 (FIG. 3) that is received in and engages with a corresponding longitudinal recess 71 (FIG. 4) formed an inner surface defining the hollow interior 49" of the control knob 26. To translationally affix the control knob 26 to the rotatable shaft 31 and hence the main body 24, the outer surface of the shaft 31 also has one or more linear slots 74 oriented perpendicularly to the longitudinal axis of the rotatable shaft 31. Each slot 74 is aligned with a respective hole 76 (FIG. 5) formed through a side of the distal end portion 47 of the control knob 26, so that a respective pin 77 may be inserted into the hole 76 and the slot 74 to couple the control knob 26 and the rotatable shaft 31.

It is understood that other embodiments of the guiding sheath assembly may provide a rotatable shaft 31 with an exposed portion for direct manipulation by a user, without the control knob 26.

As shown in FIG. 3 and FIG. 5, the shuttles 32A and 32B have a similar construction to each other, with the understanding that each is generally a mirror image of the other, although the first shuttle 32A is driven by the rotatable shaft 31 and second shuttle 32B is driven by the first shuttle 32A via the pinion 34 situated between them. Each shuttle 32A and 32B has a respective elongated body having a distal portion 80A and 80B with a C-shaped end cross-section, and a respective proximal rack portion 90A and 90B with a respective plurality of teeth 92A and 92B arranged longitudinally. The first and second shuttles are arranged to face each other and engage the pinion 34 such that the distal portions 80A and 80BC together can form a cylindrical form with an outer circumferential surface that fits within the threaded passage 42, and an inner circumferential surface that defines a passage 93 for the guiding sheath 12 to pass through. As shown in FIG. 5, the rack portion 90A and 90B of each shuttle faces each other with the pinion 34 in between so that the teeth 92A and 92B of each rack portion can engage with teeth of the pinion 34 which is mounted for rotation about an axis perpendicular to the longitudinal axis 55 of the control handle 16.

With reference to FIG. 2 and FIG. 3, an outer surface of the distal portion 80A of the first shuttle 32A is configured with an external or male threaded surface 85. An inner circumferential surface of the rotatable shaft 31 is configured with an internal or female threaded surface 86 (FIG. 4) which receives the male threaded surface 85 of the first shuttle 32A for coupling the first shuttle 32A and the rotatable shaft 31 in converting rotational movement of the rotatable shaft 31 into translation movement of the first shuttle 32A. In contrast, the outer surface of the distal portion 80B of the second shuttle 32B is smooth, without any feature engaging the threaded female surface of the rotatable, so that it can move independently of the threaded male surface 85. Accordingly, as a user rotates the control knob 26 in a first direction, the rotatable shaft 31 which is rotationally coupled to the control knob 26 via the longitudinal ridge 70 also rotates. With the rotatable shaft 31 rotationally and translationally locked to the control knob 26 via the longitudinal ridge 70 and the one or more pins 77, rotation of the shaft 31 drives the first shuttle 32A to translate along the longitudinal axis in a first direction (for example, proximally). As the first shuttle 32A translates, its teeth 92A drive the pinion 34 to rotate in a first direction (for example, clockwise), which in turn drives the second shuttle 32B to translate along the longitudinal axis 55 in a second direction opposite of the first direction (for example, distally). So arranged, the male and female threaded surfaces 85 and 86 convert rotational movement of the control knob 26 into linear movement of the shuttles 32A and 32B. With proximal ends of the first and second puller wires 30A and 30B anchored, coupled or otherwise responsive to the first and second shuttles 32A and 32B, respectively, linear and opposite movements of the shuttles actuate the puller wires for bi-directional deflection of the deflection section 14 of the guiding sheath 12. In the illustrated embodiment, the proximal ends of the puller wires 30A and 30B are coupled to the rack portions 90A and 90B of the shuttles 32A and 32B, respectively. Thus, when one puller wire is drawn proximally under tension by its respective shuttle, the other puller wire is simultaneously released from tension by its respective shuttle moving distally.

As shown in FIG. 2, a proximal end segment of each puller wire 30A and 30B extends outside of the sheath 12, in a respective longitudinal channel 88A and 88B formed in the proximal rack portion 90A and 90B of each shuttle 32A and 32B. As shown in FIG. 5, a stop 89A and 89B, for example, a hypotube, is affixed to the proximal end of each puller wire 30A and 30B, and the stop is positioned proximal of a proximal end 87A and 87B of the respective rack portion 90A and 90B so that the rack portion can push or otherwise act on the stop 89A and 89B, respectively, to draw the puller wire 30A and 30B proximally when the shuttle 32A and 32B is moved proximally. When a shuttle is moved distally, the proximal end of the rack portion comes out of contact with the respective stop, releasing the puller wire from tension. It is understood that the stop 89A and 89B may also be embedded or otherwise anchored to the respective rack portion or any part of the shuttle to effect deflection of the sheath.

Because the first and second shuttles 32A and 32B move in opposite directions along the longitudinal axis 55, an initial positioning of the shuttles relative to each other and to the passage 42 is made during assembly of the control handle. For example, as shown in FIG. 2, each shuttle is positioned in the passage 42 of the rotatable shaft 31 such that they are even with each other along the longitudinal axis 55, and each has a distal end positioned generally at mid-point along the passage 42 so that each shuttle has sufficient room to move correspondingly proximally or distally within the rotatable shaft 31. The stops 89A and 89B may be positioned relative to the shuttles such that there is minimal or even tension exerted on each puller wire 30A and 30B for a generally neutral guiding sheath with little, if any, deflection. So arranged, the shuttles adopt a "neutral" or initial configuration from which the user may evenly deflect the guiding sheath bidirectionally.

As shown in FIG. 5, the pinion 34 is positioned in between and relative to the shuttles 32A and 32B so that their teeth 92A and 92B remain engaged while the shuttles translate in response the user's manipulation of the control knob 26. In that regard, the length of the rack portions 90A and 90B are sufficiently long to ensure such continuous engagement.

It is understood that by changing one or more factors, including, for example, the length of the passage 42, the length of each distal portion 80A and 80B, the length of the rack portion 90A and 90B, the position of the pinion 34, and the number of pinions, different shuttle movement and deflection characteristics and limitations may be achieved, as needed or desired.

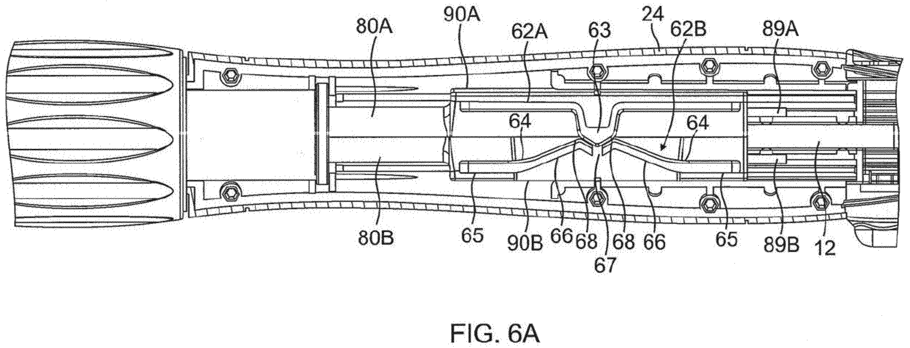

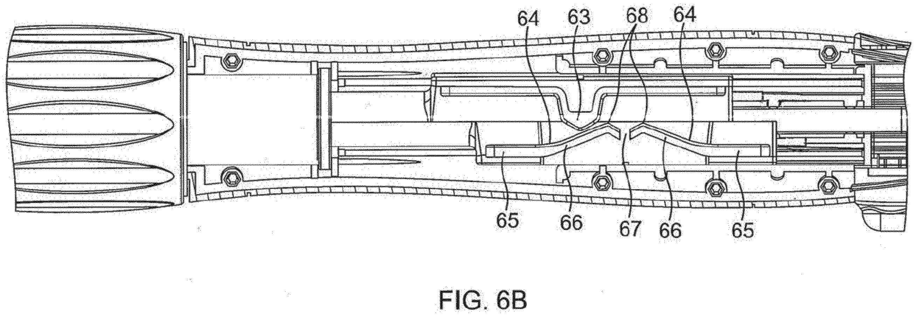

With reference to FIG. 6A and FIG. 6B, an outer surface of each rack portion 90A and 90B, opposite of the teeth 92A and 92B, of each shuttle 32A and 32B is configured with a neutral indicator. The neutral indicator includes a first member 62A and a second member 62B configured for releasable engagement with each other to indicate a neutral position between the first and second shuttles 32A and 32B, that is, a relative position where the puller wires 30A and 30B are neutral and the guiding sheath 12 accordingly is generally straight, without deflection. In the illustrated embodiment, the first or male member 62A formed on the first shuttle 32A has a tapered projection 63 facing the second or female member 62B formed on the second shuttle 32B, which includes a pair of flexible guide rails 64 on either side, whose fixed ends 65 are affixed to the second shuttle 32B and whose free ends 66 are configured to jointly form a tapered recess 67 in which the tapered projection 63 nests when the shuttles 32A and 32B are in the neutral configuration.

Accordingly, the user is typically initially presented with the guiding sheath 12 undeflected where the first and second shuttles 32A and 32B are even with each other with the tapered projection 63 nesting in the tapered recess 67, as shown in FIG. 6A. When the user rotates the control knob 26 in one direction which drives the first and second shuttles 32A and 32B to translate in opposition directions, as shown in FIG. 6B, the tapered projection 63 disengages and moves out from the tapered recess 67 but only when the user rotates the control knob with sufficient force to flex the guide rails 64 and overcome the resistance presented by their angled ends 68. When the tapered projection 63 rides over and has moved past one of the angled ends 68, the guide rail 64 is sloped such that the resistance to movement of the tapered projection 63 decreases as the tapered projection 63 moves further away from the tapered recess 67. Thus, in rotating the control knob 26 to deflect the guiding sheath 12, the user experiences a greater or maximum resistance when the shuttles 32A and 32B initially move out of the neutral configuration, followed by increasing ease as the shuttles 32A and 32B translate in opposite directions. The control handle 16 may bear visual and/or tactile indicia to provide constant orientation of deflection direction. For example, clockwise rotation of the control knob 26 consistently deflects the shaft 12 toward the side or direction of the sideport 21, and counterclockwise rotation of the control knob 26 consistently deflects the shaft 12 toward an opposite side or direction.

Conversely, when releasing the deflection of the guiding sheath 12, the user rotates the control knob 26 in the opposite direction. As the shuttles 32A and 32B translate and approach each other and begin to laterally realign again, the tapered projection 63 and the tapered recess 67 approach each other and the user applies an increasing force to rotate the control knob 26 in order for the tapered projection 63 to ride back over the angled end 68 of a guide rail 64 before the tapered projection 63 can nest in the tapered recess 67. Accordingly, the increasing resistance posed by either of the sloped rails 64 and a greater or maximum resistance posed by an angled end 68 provides the user with a tactile feel or indication of when the tapered projection 63 is in the immediate proximity of the tapered recess 67. Engagement of the tapered projection 63 and the tapered recess 67 can provide the user with an audible "click" or signal when the flexible guide rail 64 snaps into its natural configuration when the tapered projection 63 no longer exerts any load on it.

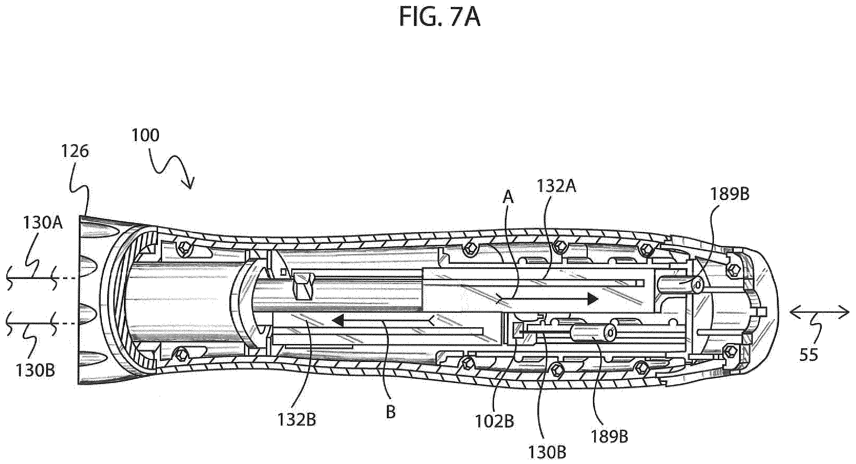

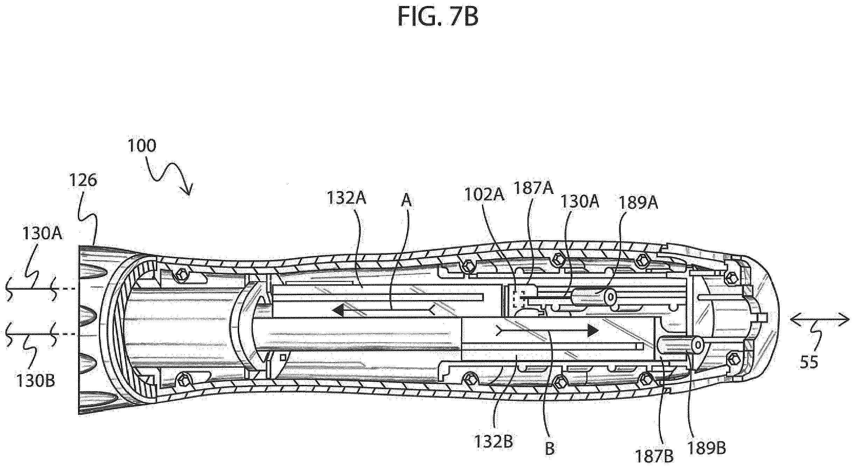

In some embodiments, as shown in **FIG. 7A** **and** **FIG. 7B****,** a control handle 100, for example for a deflectable guiding sheath, includes stops 189A, 189B affixed to proximal ends of puller wires 130A, 130B, respectively, where the stops 189A, 189B are situated proximally of proximal ends 187A, 187B of shuttles 132A, 132B so that selective movement of the shuttles by a user manipulating control knob 126 acts on the stops 189A, 189B to deflect a flexible guiding shaft (see, e.g. 12 in Fig. 1) that is distal of the control handle 100 and through which the puller wires extend. In that regard, each shuttle 132A, 132B has a corresponding deflection sensor 102A, 102B (shown in broken lines) configured to detect at least the occurrence of deflection, if not also the amount, extent or degree of deflection of the guiding shaft.

In FIG. 7A, the control knob 126 has been manipulated (e.g., turned about the longitudinal axis 55) by the user to translate the shuttle 132A proximally (arrow A) while translating the shuttle 132B distally (arrow B). In particular, the proximal end 187A pushes against the stop 189A moving the stop proximally while the proximal end 187B moves distally releasing the stop 189B. As such, the puller wire 130A is drawn tautly in the proximal direction to deflect the guiding sheath toward its side of the control handle 126 while the puller wire 130B is free to move distally to allow such deflection.

In FIG. 7B, the control knob 126 has been manipulated (e.g., turned about the longitudinal axis 55) by the user to translate the shuttle 132A distally (arrow A) while translating the shuttle 132B proximally (arrow B). In particular, the proximal end 187B pushes against the stop 189B moving the stop proximally while the proximal end 187A moves distally releasing the stop 189A. As such, the puller wire 130B is drawn tautly in the proximal direction to deflect the guiding sheath toward its side of the control handle 126 while the puller wire 130A is free to move distally to allow such deflection.

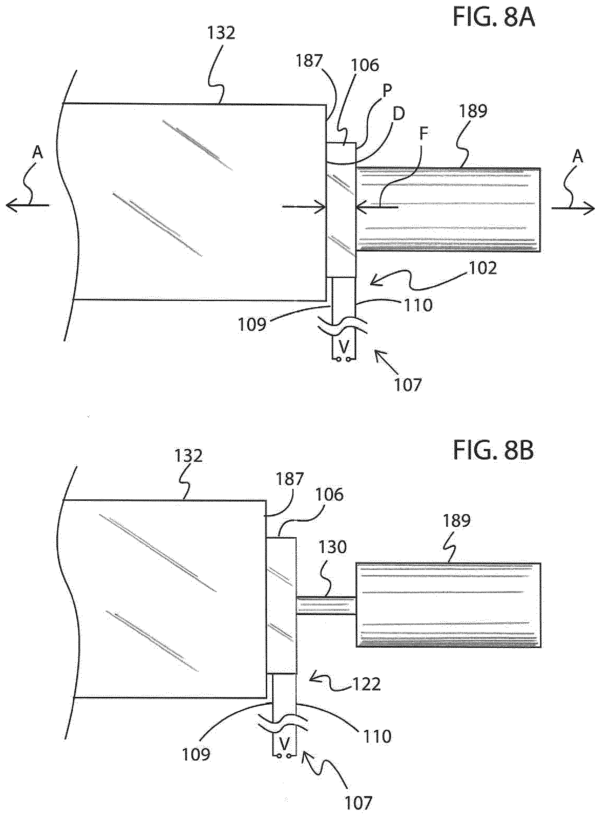

In some embodiments, each deflection sensor 102 includes a respective piezoelectric pressure sensor which, as understood by one ordinary skill in the art, is responsive to applied pressure in generating a voltage that is proportional to the applied pressure. In the illustrated embodiment of **FIG. 8A** and **FIG. 8B****,** the piezoelectric pressure sensor includes a piezoelectric element 106 and corresponding voltmeter circuit 107. The piezoelectric element, which may be, for example, a crystal, is positioned between the stop 189 and the proximal end 187 of the shuttle so that actuation of the shuttle results in the compression of the piezoelectric element 106. When a compressive force F (FIG. 8A) is applied to the piezoelectric element, an electric charge is generated across the faces of the piezoelectric element 106 as understood by one of ordinary skill in the art. The electric charge can be measured as a voltage V in the circuit 107 proportional to the compressive force. Advantageously, no external voltage or current source is required. Each piezoelectric element 106 generates an output signal directly from the applied compressive pressure, which is measured by the voltmeter circuit including two leads 109, 110 for each piezoelectric element.

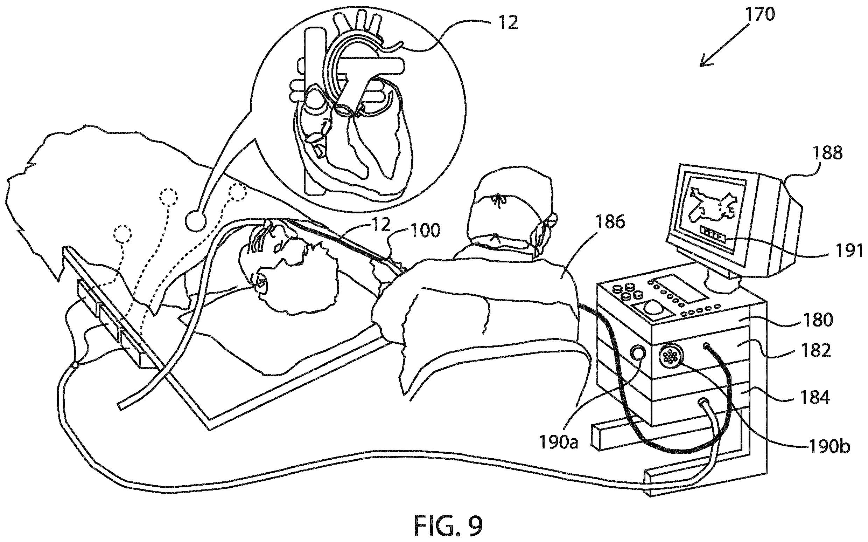

In some embodiments, as illustrated in **FIG. 8A****,** the voltage V representative of the amount of compressive force acting on the piezoelectric element 106 is measured across a distal end D and proximal end P of each piezoelectric element 106, that is, longitudinally across the piezoelectric element in a direction generally parallel with the longitudinal axis A of the control handle 100, as the compressive force exerted by the shuttle 132 on the piezoelectric element 106 between the stop 189 and the proximal end 187 of the shuttle is the greatest in the direction of the longitudinal axis A. Accordingly, the lead 109 is connected to the distal end D of the piezoelectric element and the lead 110 is connected to the proximal end P of the piezoelectric element. As understood by one of ordinary skill in the art, the leads 108 and 109 may extend proximally through the control handle to another location, for example, more proximally at a remote signal processing location in an electrophysiology system 170 that includes a controller 180, as shown in **FIG. 9****,** with a processor 182 configured to process output signals from the deflection sensor. The piezoelectric pressure sensor is particularly sensitive to dynamic changes in compressive force and is thus particular suitable for measuring small changes in compressive force.

In some embodiments, the degrees or curvatures of deflection of guiding shaft 112 distal of the control handle 100 that are actuated by the shuttles 132A, 132B in response to manipulated of the control knob 126 by a user are calibrated to different voltages that are generated by the piezoelectric elements 106A, 106B in response to the amount of compressive force exerted on the piezoelectric element between the respective shuttle and stop when the shaft 112 is deflected. Thus, when the catheter is in use in a procedure on a patient, an index correlating measured voltage and degree or curvature of deflection may be stored in a memory 184 of the processor 182 of the console 180 and referenced to provide a user 186 with an output of a degree or curvature of deflection based on an input of measured voltage derived from the voltmeter circuit of the deflection sensor. In general, the higher the measured voltage V, the greater the compressive force and thus the greater the degree or curvature of the deflection of the guiding shaft in the selected direction. In some embodiments, a minimum voltage output by the deflection sensor is 0.0 V and a maximum voltage output is approx. 5.0 V. In some embodiments, the maximum voltage output is approx. 10 V. In some embodiments, the maximum voltage output is approx. 20 V.

In some embodiments, the electrophysiology system 170 includes a display 188 configured to respond to the processor 182 in displaying a voltage indicator or reading 191 to a user. In some embodiments, the system 170 includes one or more deflection indicator, for example, an LED 190a and a speaker 190b, configured to be activated by the processor 58 to provide an audio and/or visual cue to the user when the voltage output of the deflection sensor exceeds a predetermined threshold which represents a maximum deflection of the shaft 12 to avoid breakage or damage to the shaft. The deflection indicators can also be configured to provide distinguishable audio and/or visual cues between left deflection, right deflection and/or neutral (no deflection) in the guiding shaft based on the identity of the deflection sensor 102A, 102B that is generating the voltage output or the absence of a voltage output by left and right deflection sensors. It is understood that the processor can also be configured to detect a rate of change of the voltage measured in determining other aspects of the deflection, such as whether deflection curve is tightening or relaxing.

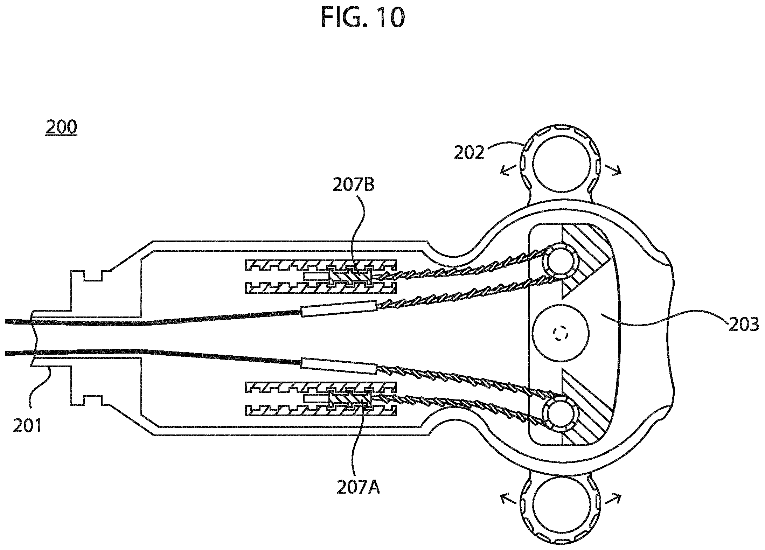

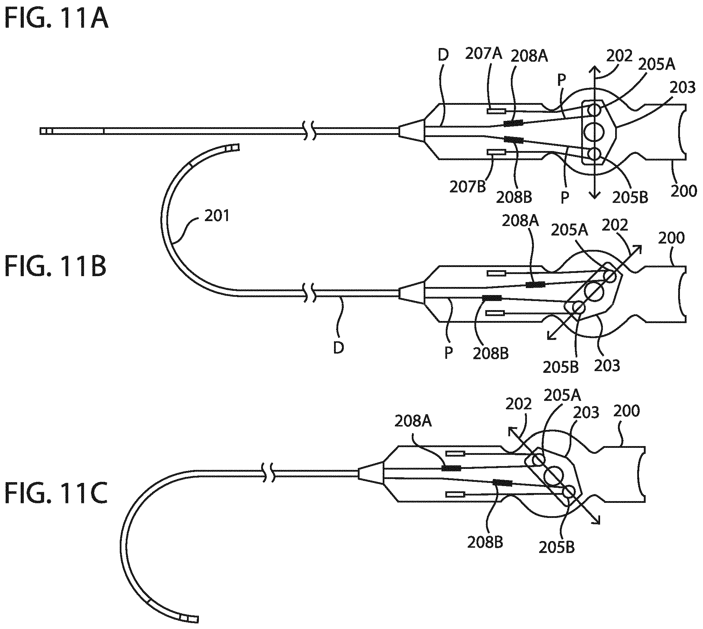

It is understood that the deflection sensors may be incorporated in any suitable control handle of an electrophysiology (EP) device with uni- or bi-directional deflection, including catheters and probes that have elements within a control handle that are movably coupled or engaged to each other such that an element is subjected to compressive or tensile forces in the deflection of a shaft. As shown in **FIG. 10****,** a control handle 200 suitable for use with an EP catheter or probe has a shaft 201 that is deflectable by a user manipulating a deflection control lever 202 which actuates a rocker 203 that draws on first and second tensile members 204A, 204B. A suitable control handle is disclosed in U.S. Patent No. 7,377,906, titled Steering Mechanism For Bi-Directional Catheter. Each tensile member includes a distal portion D (e.g., puller wire) that extends distally into the shaft 201, and a proximal portion P (e.g., spun fibers such as VECTRAN^{®}) that is wrapped around a respective pulley 205A, 205B of the rocker 203, with a proximal end that is affixed to a respective stop 207A, 207B anchored in housing of the control handle. The distal and proximal portions D and P of each tensile member are connected by a respective connector 208A, 208B, e.g.,a ferrule. As understood by one of ordinary skill in the art, when the user rotates the deflection control lever 202, the rocker 203 rotates with the lever which draws one pulley proximally and moves the other pulley distally, as shown in **FIG. 11A, FIG. 11B** and **FIG. 11C****.** The drawn pulley draws the proximal portion P which exerts a force, including a strain force, on the ferrule 208A.

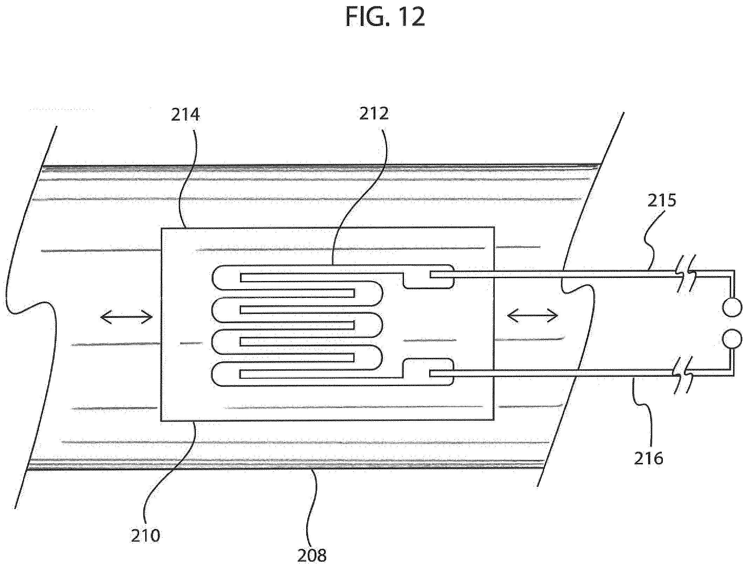

A strain gauge 210 mounted on the surface of each ferrule 208, as shown in **FIG. 12****,** detects the deformation of the ferrule to produce a shift in electrical resistance in the strain gauge that is proportional to the strain applied to the surface. In some embodiments, the strain gauge 210 includes a winding pattern of etched metal wire 212 (e.g., a copper-nickel alloy) on a substrate 214, (e.g., a flexible polyimide film). As understood by one of ordinary skill in the art, the strain gauge changes its resistance due to geometry changes. In particular, when the metal wire experiences a tensile strain, the wire is elongated and its cross-sectional area decreases which jointly contribute to an increase in the resistance of the wire. Likewise, when the wire experiences a compressive strain, the wire is shortened and its cross-sectional area increases which jointly contribute to a decrease in the resistance of the wire. When bonded to a surface of the connector (e.g., the ferrule 208), the strain gauge 210 subjected to the strain force experienced by the connector when pulled on the pulley. The strain gauge flexes and distorts in unison with the surface of the connector causing a shift in the electrical resistance of the strain gauge that is proportional to the strain applied to the surface. The strain is measured by the voltmeter circuit that include leads 215 and 216 complete the voltmeter circuit. As understood by one of ordinary skill in the art, the leads 215 and 216 may extend proximally through the control handle to another location, for example, more proximally at a remote signal processing location in the electrophysiology system 170 that includes the console 180 with the processor 182 configured to process output signals from the deflection sensor, with similar indexing of different outputs with different deflection curvatures, as described above.

It is understood that strain gauges 210 may also be affixed to surface of the stops 207A and 207B respectively, to detect deformation of the stops when the stops are subjected to deformation when the rocker arm is manipulated to deflect the catheter shaft.

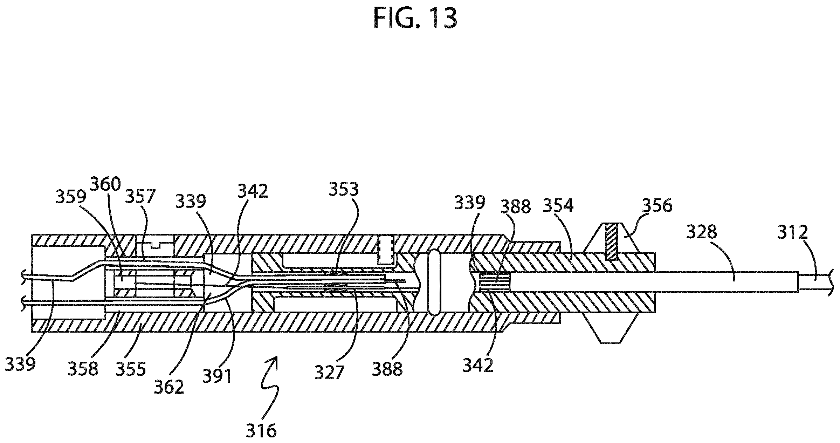

The aforementioned deflection sensor may also be incorporated in a uni-directional control handle. A suitable uni-directional deflection control handle is described in U.S. Patent No. 6,602,242, titled Irrigated Tip Catheter. As shown in **FIG. 13****,** longitudinal movement of the puller wire 342 relative to the catheter body 312, which results in deflection of the tip section (not shown), is accomplished by suitable manipulation of a control handle 316. The distal end of the control handle 316 comprises a piston 354 with a thumb control 356 for manipulating the puller wire 342. The proximal end of the catheter body 312 is connected to the piston 354 by means of a shrink sleeve 328.

The puller wire 342 and other components, e.g., lead wires, thermocouple wires, and first infusion tube segment 388 extend through the piston 354. The puller wire 342 is anchored to an anchor pin 357, located proximal to the piston 354. Within the control handle 16, the lead wires and thermocouple wires are within the protective sheath 339. Within the piston 354, the first infusion tube segment 388 extends into another protective sheath 391, preferably made of polyurethane. The protective sheathes 339 and 391 are anchored to the piston 354, preferably by polyurethane glue or the like at a glue joint 353, allowing the first infusion tube segment 388, lead wires and thermocouple wires longitudinal movement within the control handle 316 so that they do not break when the piston 354 is adjusted to manipulate the puller wire 342. Within the piston 354, the puller wire 342 extends through a transfer tube 327, preferably a polyimide tube, to allow longitudinal movement of the puller wire near the glue joint 353.

The piston 354 lies within the barrel 355 of the control handle. The barrel 355 is generally solid having a piston chamber for receiving the piston 354. Extending proximally from the piston chamber are three longitudinal holes 358, 359 and 360 and a transverse hole for receiving the anchor pin 357. The second longitudinal hole 359 is in communication with the transverse hole. The first infusion tube segment 388 within the protective sheath 391 extends through the first longitudinal hole 358. The puller wire 342 extends through the second longitudinal hole 359 and is anchored to the anchor pin 357 in the transverse hole. The thermocouple wires and lead wires within the protective sheath 339 extend through the third longitudinal hole 360. Between the distal end of the longitudinal holes 358, 359 and 360 and the proximal end of the piston 354, chamber 362 provides additional space to avoid undesirable bending of the first infusion tube segment 388.

In some embodiments, the puller wire 342 includes a distal portion and a proximal portion that are connected by a connector, e.g., the ferrule 208 of **FIG. 12****,** situated in the barrel 355, wherein the strain gauge 210, described above, is positioned on the connector and subjected to the tensile force experienced by the ferrule 208 when drawn on by the piston 354. In some embodiments, the strain gauge is affixed to anchor pin 357 to detect strain and deformation of the anchor to which the proximal end of the puller wire is attached.

The preceding description has been presented with reference to presently preferred embodiments of the invention. Workers skilled in the art and technology to which this invention pertains will appreciate that alterations and changes in the described structure may be practiced without meaningfully departing from the scope of this invention, defined by the claims. Within the invention defined by the claims, any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. An electrophysiology system (170), comprising:
a guiding sheath assembly (10), comprising:
an elongated shaft (12);
a control handle (100) proximal of the shaft, the control handle having a longitudinal axis (55) and a deflection control knob (126), and including a shuttle (132A, 132B) configured for translation along the longitudinal axis in response to manipulation of the control knob;
a puller wire (130A, 130B) extending along the shaft and responsive to translation of the shuttle to deflect the elongated shaft, the puller wire having a stop (189A, 189B) at its proximal end; and
a deflection sensor (102A, 102B) configured to generate a signal in response to compression between the shuttle and the stop in when the elongated shaft is deflected; and
a controller (180) having a processor (182) configured to receive the signal and to perform the acts of:
measuring a voltage from the signal; and
determining an occurrence of deflection based on the voltage measured.

2. The system of claim 1, wherein the processor is further configured to perform the act of determining a degree of deflection based on the voltage measured.

3. The system of claim 2, wherein the controller includes a memory (184) configured to store an index correlating predetermined voltages and predetermined degrees of deflection, and the processor is further configured to access the index to determine the degree of deflection based on the voltage measured.

4. The system of claim 1, wherein the deflection sensor includes a piezoelectric pressure sensor (106A 106B).

5. The system of claim 1, further including an indicator (190a, 190b) responsive to the controller and configured to provide a cue to a user indicative of the occurrence of deflection based on the voltage measured.

6. The system of claim 5, wherein the indicator is configured to provide a visual cue.

7. The system of claim 5, wherein the indicator is configured to provide an audio cue.

8. An electrophysiology catheter, comprising:
an elongated shaft (201);
a control handle (200) proximal of the shaft, the control handle having a longitudinal axis and a control knob (202), and including:
a rocker (203) responsive to the control knob to deflect the elongated shaft, the rocker having a pulley member (205A, 205B);
a tensile member (204A, 204B) having a distal portion extending through the shaft and a proximal portion extending around the pulley member;
a connector (208A, 208B) extending between the distal and proximal portions of the tensile member; and
a strain gauge (210) affixed to the connector and configured to generate a signal in response to strain of the connector.

## Patentansprüche

1. Elektrophysiologisches System (170), umfassend:
eine Führungshülsenanordnung (10), umfassend:
eine längliche Welle (12);
einen Steuergriff (100) proximal zu der Welle, wobei der Steuergriff eine Längsachse (55) und einen Auslenkungssteuerknopf (126) aufweist und ein Schiffchen (132A, 132B) einschließt, das für eine Verschiebung entlang der Längsachse unter Ansprechen auf eine Manipulation des Steuerknopfs konfiguriert ist;
einen Zugdraht (130A, 130B), der sich entlang der Welle erstreckt und auf die Verschiebung des Schiffchens anspricht, um die längliche Welle auszulenken, wobei der Zugdraht einen Anschlag (189A, 189B) an seinem proximalen Ende aufweist; und
einen Auslenkungssensor (102A, 102B), der konfiguriert ist, um ein Signal unter Ansprechen auf eine Kompression zwischen dem Schiffchen und dem Anschlag zu erzeugen, wenn die längliche Welle ausgelenkt wird; und
eine Steuerung (180), die einen Prozessor (182) aufweist, der konfiguriert ist, um das Signal zu empfangen und um die Handlungen durchzuführen zum:
Messen einer Spannung von dem Signal; und
Bestimmen eines Auftretens einer Auslenkung basierend auf der gemessenen Spannung.

2. System nach Anspruch 1, wobei der Prozessor ferner konfiguriert ist, um die Handlung des Bestimmens eines Auslenkungsgrads basierend auf der gemessenen Spannung durchzuführen.

3. System nach Anspruch 2, wobei die Steuerung einen Speicher (184) einschließt, der konfiguriert ist, um einen Index zu speichern, der zuvor bestimmte Spannungen und zuvor bestimmte Auslenkungsgrade korreliert, und der Prozessor ferner konfiguriert ist, um auf den Index zuzugreifen, um den Auslenkungsgrad basierend auf der gemessenen Spannung zu bestimmen.

4. System nach Anspruch 1, wobei der Auslenkungssensor einen piezoelektrischen Drucksensor (106A 106B) einschließt.

5. System nach Anspruch 1, ferner umfassend eine Anzeige (190a, 190b), die auf die Steuerung anspricht und konfiguriert ist, um einem Benutzer einen Hinweis, der das Auftreten der Auslenkung basierend auf der gemessenen Spannung anzeigt, bereitzustellen.

6. System nach Anspruch 5, wobei die Anzeige konfiguriert ist, um einen visuellen Hinweis bereitzustellen.

7. System nach Anspruch 5, wobei die Anzeige konfiguriert ist, um einen Audiohinweis bereitzustellen.

8. Elektrophysiologischer Katheter, umfassend:
eine längliche Welle (201);
einen Steuergriff (200) proximal zu der Welle, wobei der Steuergriff eine Längsachse und einen Steuerknopf (202) aufweist und einschließt:
eine Wippe (203), die auf den Steuerknopf anspricht, um die längliche Welle auszulenken, wobei die Wippe ein Rollenelement (205A, 205B) aufweist;
ein zugbelastbares Element (204A, 204B), das einen distalen Abschnitt, der sich durch die Welle erstreckt, und einen proximalen Abschnitt, der sich um das Rollenelement herum erstreckt, aufweist;
einen Verbinder (208A, 208B), der sich zwischen den distalen und proximalen Abschnitten des zugbelastbaren Elements erstreckt; und
einen Dehnungsmessstreifen (210), der an dem Verbinder befestigt und konfiguriert ist, um ein Signal unter Ansprechen auf eine Dehnung des Verbinders zu erzeugen.

## Revendications

1. Système d'électrophysiologie (170), comprenant :
un ensemble de gaine de guidage (10), comprenant :
une tige allongée (12) ;
une poignée de commande (100) proximale à la tige, la poignée de commande ayant un axe longitudinal (55) et un bouton de commande de déviation (126), et comportant une navette (132A, 132B) conçue pour se déplacer le long de l'axe longitudinal en réponse à une manipulation du bouton de commande ;
un fil de traction (130A, 130B) s'étendant le long de la tige et réagissant à la translation de la navette pour dévier la tige allongé, le fil de traction ayant une butée (189A, 189B) au niveau de son extrémité proximale ; et
un capteur de déviation (102A, 102B) configuré pour générer un signal en réponse à la compression entre la navette et l'arrêt lorsque la tige allongé est dévié ; et
un dispositif de commande (180) ayant un processeur (182) configuré pour recevoir le signal et pour effectuer les actions consistant à :
mesurer une tension à partir du signal ; et
déterminer une occurrence de déviation sur la base de la tension mesurée.

2. Système selon la revendication 1, dans lequel le processeur est en outre configuré pour effectuer l'action de détermination d'un degré de déviation sur la base de la tension mesurée.

3. Système selon la revendication 2, dans lequel le dispositif de commande comporte une mémoire (184) configurée pour stocker un indice mettant en corrélation des tensions prédéterminées et des degrés de déviation prédéterminés, et le processeur est en outre configuré pour accéder à l'indice afin de déterminer le degré de déviation sur la base de la tension mesurée.

4. Système selon la revendication 1, dans lequel le capteur de déviation comporte un capteur de pression piézoélectrique (106A, 106B).

5. Système selon la revendication 1, comportant en outre un indicateur (190a, 190b) réagissant au dispositif de commande et configuré pour fournir un indice à un utilisateur indiquant l'occurrence d'une déviation sur la base de la tension mesurée.

6. Système selon la revendication 5, dans lequel l'indicateur est configuré pour fournir un signal visuel.

7. Système selon la revendication 5, dans lequel l'indicateur est configuré pour fournir un signal audio.

8. Cathéter électrophysiologique comprenant :
une tige allongée (201) ;
une poignée de commande (200) proximale à la tige, la poignée de commande ayant un axe longitudinal et un bouton de commande (202), et comportant :
une culbuteur (203) réagissant au bouton de commande pour dévier la tige allongé, le culbuteur ayant un élément de poulie (205A, 205B) ;
un élément de traction (204A, 204B) ayant une partie distale s'étendant à travers la tige et une partie proximale s'étendant autour de l'élément de poulie ;
un connecteur (208A, 208B) s'étendant entre les parties distale et proximale de l'élément de traction ; et
une jauge de contrainte (210) fixée au connecteur et configurée pour générer un signal en réponse à une contrainte du connecteur.
